# EUROPÄISCHE PATENTSCHRIFT

(11) **EP 1 004 288 B1**
(45) Veröffentlichungstag und Bekanntmachung des Hinweises auf die Patenterteilung: **19.06.2002**
(21) Anmeldenummer: 99120518.8
(22) Anmeldetag: 15.10.1999
(51) Int. Cl.: A61K 7/00, A61K 7/11, A61K 7/13

(54) **Pigmenthaltiges, verschäumbares Gel**
Pigment containing, foamable gel
Gel moussant contenant des pigments

(30) Priorität: 28.11.1998 DE 19855097
(43) Veröffentlichungstag der Anmeldung: 31.05.2000
(73) Patentinhaber: Wella Aktiengesellschaft, 64274 Darmstadt (DE)
(72) Erfinder: Franzke, Michael, 64380 Rossdorf (DE); Stein, Bernd, 63768 Hösbach (DE)

(56) Entgegenhaltungen:
- EP-A- 0 172 713
- EP-A- 0 887 067
- EP-A- 0 978 271
- DE-U- 29 813 861
- US-A- 5 833 968
- CHEMICAL ABSTRACTS, vol. 109, no. 6, 8. August 1988 (1988-08-08) Columbus, Ohio, US; abstract no. 43304, TERAMOTO, KEIICHIRO: "Aerosol foam compositions containing titanium mica, carboxyvinyl polymer, and anionic resins for coloring hair" XP002130987 & JP 62 255414 A (OSAKA AEROSOL INDUSTRY CO., LTD., JAPAN) 7. November 1987 (1987-11-07)

## Beschreibung

Gegenstand der Erfindung ist ein verschäumbares, pigmenthaltiges Gel.

Die Erzeugung von Farbeffekten auf Haaren ist auf verschiedene Weise möglich. Bei der Verwendung von Oxidationshaarfarben werden Farbstoffvorstufen verwendet, die in das Haar eindringen und dort durch Oxidationsreaktionen den Farbstoff bilden. Diese Färbungen sind dauerhaft und können nicht ohne weiteres ausgewaschen werden. Eine weitere Möglichkeit zur Färbung von Haaren ist die Verwendung der sogenannten direktziehenden Farbstoffe. Hierbei handelt es sich um lösliche, organische Farbstoffe, welche auf das Haar aufziehen. Auch diese Färbungen sind relativ dauerhaft und überstehen mehrere Haarwäschen. Häufig sollen Farbeffekte aber nicht dauerhaft, sondern leicht aufzubringen und ebenso leicht wieder zu entfernen sein. Ein Weg, um dies zu erreichen, ist die Verwendung von Pigmenten. Pigmente sind unlösliche farbige Materialien, die sich auf dem Haar ablagern können. Farbeffekte mit Pigmenten weisen häufig den Nachteil auf, daß sie eine zu geringe Abriebfestigkeit besitzen. An pigmenthaltige Haarbehandlungsmittel sind eine Reihe von Anforderungen zu stellen: eine ausreichende Haftung der Pigmente am Haar, eine möglichst große Abriebfestigkeit gegen mechanischen Abrieb, eine leichte Entfernbarkeit durch eine Haarwäsche und eine hohe Farbbrillanz der behandelten Haare. Weiterhin soll das verwendete Mittel, insbesondere, wenn es sich um ein Farbstylingmittel handelt, dem Haar eine gute Formbarkeit und guten Halt verleihen. Gleichzeitig soll das Mittel in einer optisch ansprechenden und leicht handhabbaren Applikationsform vorliegen.

Haarbehandlungsmittel in Schaumform zum Färben von Haaren mit Pigmenten sind aus der EP 0 172 713 bekannt. Bei diesen Färbemitteln handelt es sich um Schaumaerosole, welche in einem nicht-viskosen Medium vorliegen. Derartige Mittel haben den Nachteil, daß sie vor der Anwendung geschüttelt werden müssen, da sich die ungelösten Pigmentteilchen am Boden des Aerosolbehälters absetzen.

In dem Abstract zur JP 62-255414 (Chemical Abstracts, vol. 109, no. 6, abstract no. 43304) wird eine verschäumbare Aerosol Zusammensetzung beschrieben, welche ein Carboxyvinylpolymer als Verdicker, ein anionisches Harz als Bindemittel und Titaniummica-Pigmente enthält. In der EP 0 887 067 A2 werden Haarkosmetika beschrieben mit einem Gehalt an mit Titandioxid beschichtetem Mica, einem Carboxyvinylpolymer und einem amphoren Makromolekül, wobei Titandioxid beschichtetes Mica mit einer Teilchengröße von 20 µm oder größer nicht mehr als 10 Volumen% bezogen auf das Gesamtvolumen von TiO2 beschichtetem Mica ausmacht. In der EP 0 978 271 A1 wird eine Aerosolschaumzusammensetzung beschrieben mit einem Gehalt an Glanzpigmenten, einem (Meth)acrylsäure-Polymerisat und Aerosoltreibmitteln.

Es bestand somit die Aufgabe, ein Mittel zur Verfügung zu stellen, mit dem sich Haare schnell und leicht Färben lassen, welches in einer leicht handhabbaren und optisch ansprechenden Form vorliegt und welches die oben genannten Anforderungskriterien bei Vermeidung der genannten Nachteile erfüllt.

Es wurde nun gefunden, daß die Aufgabe gelöst wird durch ein verschäumbares, transparentes, pigmenthaltiges Gel der nachfolgend beschriebenen Zusammensetzung.

Gegenstand der Erfindung ist ein verschäumbares, farbiges Gel mit einem Gehalt an
(A) 35 bis 98 Gewichtsprozent, vorzugsweise 55 bis 95 Gewichtsprozent Wasser,
(B) mindestens einem gelbildenden Verdicker, ausgewählt unter Verdickern, die der Zusammensetzung eine Fließgrenze verleihen,
(C) mindestens einem Pigment,
(D) mindestens einem filmbildenden, haarfestigenden Polymer,
(E) mindestens einem schaumerzeugenden Tensid und
(F) mindestens einem Treibmittel
in Kombination mit einer geeigneten Aerosolverpackung und einer Vorrichtung zum Verschäumen des Gels, wobei die Konzentration des Verdickers so gewählt ist, daß einerseits die Viskosität des Gels gering genug ist, um ein Verschäumen des Gels zu ermöglichen und andererseits die Fließgrenze mindestens so groß ist, daß ein Absinken der Pigmente verhindert ist, ausgenommen einer Zusammensetzung enthaltend (a) Titandioxid beschichtetes Mica, wobei Titandioxid beschichtetes Mica mit einer Teilchengröße von 20 µm oder größer nicht mehr als 10 Volumen% bezogen auf das gesamte Volumen von Titandioxid beschichtetem Mica ausmacht, (b) ein Carboxyvinylpolymer und (c) ein amphoteres Makromolekül.

Als Komponente (B) kommen insbesondere Polymere in Betracht, die der Zusammensetzung ein plastisches oder pseudoplastisches Verhalten verleihen. Das rheologische Fließverhalten des erfindungsgmäßen Gels ist durch die Existenz einer Fließgrenze charakterisiert, welche vorzugsweise mindestens 3 Pascal beträgt, gemessen mit einem Haake-Rotationsviskosimeter RV 12, Meßsystem PKV-0.5 bei 30 °C und einem linear steigenden Schergefälle von 0 bis 100 s⁻¹.

Es handelt sich um einen temporären Farbfestiger, der in der Farbbrillanz, Auswaschbarkeit und Abriebfestigkeit die gestellten Anforderungen in bester Weise erfüllt. Das Mittel färbt temporär und festigt das Haar gleichzeitig. Es gibt dem Haar eine hohe Farbbrillanz und läßt es glänzend erscheinen, ist nach Belieben wieder auswaschbar und besitzt eine hohe Abriebfestigkeit. Das Mittel liegt in einer optisch ansprechenden, stabil dispergierten Form vor, was die Abpackung in einem transparenten Behälter, beispielsweise aus Glas oder durchsichtigem Kunststoff wie z.B. Polyethylenterephtalat ermöglicht. Die Pigmentteilchen sind stabil dispergiert und setzen sich auch bei langer Lagerung nicht ab. Deshalb braucht das Mittel zur Redispergierung der Pigmente vor der Anwendung nicht geschüttelt zu werden und braucht auch keine Kugel zur Unterstützung der Redispergierung der Pigmenteilchen zu enthalten. Ein weiterer Vorteil des erfindungsgemäßen Gels liegt darin, daß der sich bildende Schaum über längere Zeit äußerst stabil ist, nicht vom Haar herabläuft, sich aber dennoch sehr leicht in das Haar einarbeiten läßt, da er bei der Einarbeitung sehr leicht zusammenbricht.

Das Gel weist vorzugsweise eine nach Überschreiten der Fließgrenze meßbare Viskosität von 200 bis 15.000 mPa s bei 25°C auf. Besonders bevorzugt liegt das Gel als Flüssiggel mit einer Viskosität von 300 bis 6000, insbesondere von 500 bis 3000 mPa s bei 25°C vor. Das Gel ist dadurch gekennzeichnet, daß es eine Fließgrenze aufweist. Die Fließgrenze ist in Abhängigkeit von Gewicht und Oberfläche der Pigmentteilchen so gewählt, daß sie mindestens so groß ist wie der durch die Pigmentteilchen ausgeübte Druck. Hierdurch wird ein Absinken der Pigmentteilchen verhindert.

Die Verdicker sind vorzugsweise in einer Menge von 0,05 bis 10, besonders bevorzugt von 0,1 bis 4 Gewichtsprozent enthalten. Die jeweilige optimale Konzentration wird in Abhängigkeit vom Verdickertyp und der Dichte der Pigmente gewählt. Geeignete Verdicker sind vernetzte oder nicht vernetzte Polyacrylsäure oder Polymethacrylsäure. Von den Verdickern, die in dem erfindungsgemäßen Mittel enthalten sein können, sind Homopolymere der Acrylsäure mit einem Molekulargewicht von 2.000.000 bis 6.000.000 zu nennen, die beispielsweise von der Firma BF Goodrich/USA unter der Handelsbezeichnung Carbopol® vertrieben werden. Als weitere Verdicker kann das erfindungsgemäße Mittel ein Acrylsäurehomopolymer mit einem Molekulargewicht von 4.000.000 enthalten, das beispielsweise von der Firma BF Goodrich unter der Handelsbezeichnung Carbopol® 940 vertrieben wird. Weitere Verdicker sind beispielsweise die von der Firma BF Goodrich unter dem Handelsnamen Carbopol® ETD 2001 oder von der Firma Protex/Frankreich unter dem Handelsnamen Modarez® V 600 PX vertriebene Acrylsäurehomopolymer, das von der Firma Hoechst/ Deutschland unter dem Handelsnamen Hostacerin® PN 73 vertriebene Polymer aus Acrylsäure und Acrylamid mit einem Molekulargwicht von 2.000.000 bis 6.000.000 und das von der Firma Alban Muller, Montreuil/Frankreich unter dem Handelsnamen Amigel® vertriebene Sclerotium Gum. Besonders bevorzugt sind die Copolymere der Acrylsäure oder der Methacrylsäure, wie sie zum Beispiel unter dem Handelsnamen Carbopol® 1342 oder Pemulen® TR1 der Firma GOODRICH, USA vertrieben werden. Weitere geeignete Verdicker sind Guar Gum, Xanthan Gum, Bentonite, Hectorite.

In einer vorteilhaften Ausführungsform können neben den genannten Verdickern, die der Zusammensetzung eine ausreichende Fließgrenze verleihen, zusätzlich auch solche Verdicker enthalten sein, die der Zusammensetzung in dem für Liquid-Gele typischen Viskositätsbereich keine ausreichende Fließgrenze verleihen. Derartige Verdicker sind insbesondere Cellulosen und Cellulosederivate wie z.B. Carboxymethylcellulose, Celluloseether und Hydroxyalkylcellulosen, beispielsweise Hydroxyethyl- oder Hydroxypropylcellulose.

Enthalten die Verdicker Säuregruppen, so sind die Säuregruppen vorzugsweise zumindest teilweise mit kosmetisch verträglichen Basen neutralisiert. Als Neutralisationsmittel können für kosmetische Zwecke geeignete organische oder anorganische Basen verwendet werden. Beispiele für Basen sind Aminoalkohole wie z.B. Aminomethylpropanol (AMP), Triethanolamin oder Monoethanolamin und Ammoniak, NaOH und andere.

Die Pigmente sind vorzugsweise in einer Menge von 0,01 bis 25 Gew.%, besonders bevorzugt in einer Menge von 5 bis 15 Gew.% enthalten.

Die Pigmente sind im Anwendungsmedium praktisch unlösliche Farbmittel und können anorganisch oder organisch sein. Auch anorganisch-organische Mischpigmente sind möglich. Bevorzugt sind anorganische Pigmente. Der Vorteil der anorganischen Pigmente ist deren ausgezeichnete Licht-, Wetter- und Temperaturbeständigkeit. Die anorganischen Pigmente können natürlichen Ursprungs sein, beispielsweise hergestellt aus Kreide, Ocker, Umbra, Grünerde, gebranntem Terra di Siena oder Graphit. Bei den Pigmenten kann es sich um Weißpigmente wie z.B. Titandioxid oder Zinkoxid, um Schwarzpigmente wie z.B. Eisenoxidschwarz, Buntpigmente wie z.B. Ultramarin oder Eisenoxidrot sowie um Fluoreszenz- oder Phosphoreszenzpigmente handeln, wobei vorzugsweise mindestens ein Pigment ein farbiges, nicht-weißes Pigment ist. Geeignet sind Metalloxide, -hydroxide und -oxidhydrate, Mischphasenpigmente, schwefelhaltige Silicate, Metallsulfide, komplexe Metallcyanide, Metallsulfate, -chromate und -molybdate sowie die Metalle selbst (Bronzepigmente).

Geeignet sind insbesondere Titandioxid (CI 77891), schwarzes Eisenoxid (CI 77499), gelbes Eisenoxid (CI 77492), rotes und braunes Eisenoxid (CI 77491), Manganviolett (CI 77742), Ultramarine (Natrium-Aluminiumsulfosilikate, CI 77007, Pigment Blue 29), Chromoxidhydrat (CI77289), Eisenblau (Ferric Ferrocyanide, CI77510), Carmine (Cochineal).

Besonders bevorzugt sind Pigmente auf Mica- bzw. Glimmerbasis welche mit einem Metalloxid oder einem Metalloxychlorid wie Titandioxid oder Wismutoxychlorid sowie gegebenenfalls weiteren farbgebenden Stoffen wie Eisenoxiden, Eisenblau, Ultramarine, Carmine etc. beschichtet sind und wobei die Farbe durch Variation der Schichtdicke bestimmt ist. Derartige Pigmente werden beispielsweise unter den Handelsbezeichnung Rona®, Colorona®, Dichrona® und Timiron® von der Firma Merck, Deutschland vertrieben.

Organische Pigmente sind beispielsweise die natürlichen Pigmente Sepia, Gummigutt, Knochenkohle, Kasseler Braun, Indigo, Chlorophyll und andere Pflanzenpigmente. Synthetische organische Pigmente sind beispielsweise Azo-Pigmente, Anthrachinoide, Indigoide, Dioxazin-, Chinacridon-, Phtalocyanin-, Isoindolinon-, Perylenund Perinon-, Metallkomplex-, Alkaliblau- und Diketopyrrolopyrrol-Pigmente.

Bei den Pigmenten handelt es sich vorzugsweise nicht um Nanopigmente. Die bevorzugte Teilchengröße beträgt 1 bis 200 µm, insbesondere 3 bis 150 µm, besonders bevorzugt 10 bis 100 µm.

Das erfindungsgemäße Gel enthält als Komponente (D) mindestens ein synthetisches oder natürliches, filmbildendes, haarfestigendes Polymer. Das haarfestigende Polymer kann anionischen, nichtionischen, kationischen oder amphoteren Charakter besitzen. Besonders bevorzugt sind nichtionische Polymere. Kationische Polymere sind nur in einer solchen maximalen Menge enthalten, in der in Gegenwart von anionischen Verdickern keine unlöslichen Komplexe gebildet werden. Die haarfestigenden Polymere können einzeln oder in einem Gemisch eingesetzt werden und sind vorzugsweise in einer Konzentration von 0,01 bis 25 Gewichtsprozent, besonders bevorzugt von 0,1 bis 15 Gewichtsprozent enthalten.

Unter filmbildenden, haarfestigenden Polymeren sollen solche Polymere verstanden werden, welche allein in 0,1 bis 5%iger wäßriger, alkoholischer oder wäßrig-alkoholischer Lösung angewandt, in der Lage sind, auf dem Haar einen Polymerfilm abzuscheiden und auf diese Weise das Haar zu festigen.

Geeignete natürliche, nichtionische filmbildende Polymere mit haarfestigender Wirkung sind verschiedene Saccharidtypen wie zum Beispiel Polysaccharide oder Gemische aus Oligo-, Mono- und Disacchariden, welche beispielsweise unter dem Handelsnamen C-PUR® von der Firma Cerestar, Brüssel/Belgien vertrieben werden. Weitere geeignete, natürliche Polymere sind chinesiches Balsamharz und Cellulosederivate, zum Beispiel Hydroxypropylcellulose mit einem Molekulargewicht von 30.000 bis 50.000 g/mol, welche beispielsweise unter der Handelsbezeichnung NISSO SL® von der Firma Lehmann & Voss/Deutschland vertrieben wird.

Geeignete synthetische, nichtionische, filmbildende, haarfestigende Polymere sind Homo- oder Copolymere, die aus Monomeren aufgebaut sind, welche keine ionischen oder ionisierbaren Gruppen im Molekül aufweisen. Geeignete nichtionische Monomere sind ungesättigte, radikalisch polymerisierbare Verbindungen, insbesondere Acryl- oder Vinylverbindungen. Geeignete nichtionische Monomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete nichtionische Polymere sind zum Beispiel Homopolymere des Vinylpyrrolidons, die beispielsweise unter den Handelsbezeichnungen LUVISKOL® K von der Firma BASF, Deutschland oder PVP-K von der Firma ISP, USA vertrieben werden, sowie Homopolymere des N-Vinylformamids, die beispielsweise unter der Handelsbezeichnung PVF von der Firma National Starch/USA vertrieben werden. Weitere geeignete synthetische filmbildende, nichtionische haarfestigende Polymere sind zum Beispiel Copolymerisate aus Vinylpyrrolidon und Vinylacetat, die beispielsweise unter den Handelsbezeichnungen LUVISKOL® VA von der Firma BASF/Deutschland vertrieben werden; Terpolymere aus Vinylpyrrolidon, Vinylacetat und Vinylpropionat, die beispielsweise unter der Handelsbezeichnung LUVISKOL® VAP der Firma BASF/Deutschland vertrieben werden; Polyacrylamide, die beispielsweise unter den Handelsbezeichnungen AKYPOMINE® P 191 von der Firma CHEM-Y/Deutschland oder SEPIGEL® 305 von der Firma SEPPIC/USA vertrieben werden; Polyvinylalkohole, die beispielsweise unter den Handelsbezeichnungen ELVANOL® der Firma Du Pont oder VINOL® 523/540 der Firma Air Products/USA vertrieben werden, sowie hochmolekulares Polyethylenglykol oder hochmolekulare Copolymere von Ethylenglykol mit Propylenglykol mit festigenden Eigenschaften, die beispielsweise unter den Handelsbezeichnungen LIPOXOL® 1000 von der Firma HÜLS AG/Deutschland, PLURACOL E 4000 von der Firma BASF/Deutschland oder UPIWAX® 20.000 von der Firma UPI vertrieben werden.

Besonders bevorzugte nichtionische Polymere sind Polyvinylpyrrolidon, Polyvinylpyrrolidon/Vinylacetat Copolymer und Styrol/Vinylpyrrolidon Copolymer.

Geeignete anionische haarfestigende Polymere sind synthetische Homo- oder Copolymere mit neutralisierbare Säuregruppen enthaltenden Monomereinheiten, welche gegebenenfalls mit Comonomeren, die keine Säuregruppen enthalten, copolymerisiert sind. Als Säuregruppen kommen Sulfonsäure-, Phosphorsäure- und Carbonsäuregruppen in Betracht, von denen die Carbonsäuregruppen bevorzugt sind. Geeignete Säuregruppen enthaltende Monomere sind beispielsweise Acrylsäure, Methacrylsäure, Crotonsäure, Maleinsäure bzw. Maleinsäureanhydrid, Aldehydocarbonsäuren oder Ketocarbonsäuren.

Nicht mit Säuregruppen substituierte Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylpyrrolidon, Vinylester, Vinylalkohol, Propylenglykol. oder Ethylenglykol, aminsubstituierte Vinylmonomere wie zum Beispiel Dialkylaminoalkylacrylat, Dialkylaminoalkylmethacrylat, Monoalkylaminoalkylacrylat und Monoalkylaminoalkylmethacrylat, wobei die Alkylgruppen dieser Monomere vorzugsweise C1- bis C7-Alkylgruppen, besonders bevorzugt C1- bis C3-Alkylgruppen sind.

Geeignete anionische Polymere sind insbesondere unvernetzte oder mit polyfunktionellen Agenzien vernetzte Homopolymere der Acrylsäure oder der Methacrylsäure, Copolymere der Acrylsäure oder Methacrylsäure mit Monomeren ausgewählt aus Acrylsäure- oder Methacrylsäureestern, Acrylamiden, Methacrylamiden und Vinylpyrrolidon, Homopolymere der Crotonsäure sowie Copolymere der Crotonsäure mit Monomeren ausgewählt aus Vinylestern, Acrylsäure- oder Methacrylsäureestern, Acrylamiden und Methacrylamiden. Ein geeignetes natürliches Polymer ist beispielsweise Schellack.

Bevorzugte Polymere mit Säuregruppen sind vernetzte oder unvernetzte Vinylacetat/Crotonsäure Copolymere, die beispielsweise in Form einer 60%igen Lösung in Isopropanol/Wasser unter der Handelsbezeichnung ARISTOFLEX® von der Firma HOECHST/Deutschland beziehungsweise von der Firma BASF unter dem Handelsnamen LUVISET® CA-66 vertrieben werden. Weitere geeignete anionische Polymere sind zum Beispiel Terpolymere aus Vinylacetat, Crotonsäure und Polyethylenoxid sowie Terpolymere aus Acrylsäure, Alkylacrylat und N-Alkylacrylamid, insbesondere Acrylsäure/Ethylacrylat/N-t-Butylacrylamid Terpolymere, wie sie unter den Handelsnamen ULTRAHOLD® 8 und ULTRAHOLD® STRONG der Firma BASF/Deutschland vertrieben werden oder Terpolymere aus Vinylacetat, Crotonat und Vinylalkanoat, insbesondere Vinylacetat/Crotonat/Vinylneodecanoat Copolymere, wie sie z.B. von der Firma National Starch unter der Handelsbezeichnung RESYN 28-2930 vertrieben werden.

Geeignete amphotere haarfestigende Polymere sind zum Beispiel Copolymere gebildet aus Alkylacrylamid, insbesondere Octylacrylamid, Alkylaminoalkylacrylat oder -methacrylat, insbesondere t-Butylaminoethylmethacrylat und mindestens zwei Monomeren ausgewählt aus Acrylsäure, Methacrylsäure oder deren Ester, wie sie zum Beispiel unter dem Handelsnamen Amphomer oder Amphomer LV-71 der Firma NATIONAL STARCH, USA erhältlich sind.

Geeignete kationische haarfestigende Polymere enthalten vorzugsweise quaternäre oder protonierbare, basische Amingruppen. Die kationischen Polymere können Homooder Copolymere sein, wobei die quaternären oder basischen Stickstoffgruppen entweder in der Polymerkette oder vorzugsweise als Substituent an einem oder mehreren der Monomeren enthalten sind. Die Ammoniumbzw. Amingruppen enthaltenden Monomere können mit nicht kationischen Monomeren copolymerisiert sein. Geeignete kationische Monomere sind ungesättigte, radikalisch polymerisierbare Verbindungen, welche mindestens eine kationische oder basische Gruppe tragen, insbesondere ammoniumsubstituierte Vinylmonomere wie zum Beispiel Trialkylmethacryloxyalkylammonium, Trialkylacryloxyalkylammonium, Dialkyldiallylammonium und quaternäre Vinylammoniummonomere mit cyclischen, kationische Stickstoffe enthaltenden Gruppen wie Pyridinium, Imidazolium oder quaternäre Pyrrolidone, z.B. Alkylvinylimidazolium, Alkylvinylpyridinium, oder Alkylvinylpyrrolidon Salze. Die Alkylgruppen dieser Monomere sind vorzugsweise niedere Alkylgruppen wie zum Beispiel C1- bis C7-Alkylgruppen, besonders bevorzugt C1- bis C3-Alkylgruppen.

Die Ammonium- bzw. Amingruppen enthaltenden Monomere können mit nicht kationischen Monomeren copolymerisiert sein. Geeignete Comonomere sind beispielsweise Acrylamid, Methacrylamid, Alkyl- und Dialkylacrylamid, Alkyl- und Dialkylmethacrylamid, Alkylacrylat, Alkylmethacrylat, Vinylcaprolacton, Vinylcaprolactam, Vinylpyrrolidon, Vinylester, z.B. Vinylacetat, Vinylalkohol, Propylenglykol oder Ethylenglykol, wobei die Alkylgruppen dieser Monomere vorzugsweise C1- bis C7-Alkylgruppen, besonders bevorzugt C1- bis C3-Alkylgruppen sind.

Geeignete Polymere mit quaternären Amingruppen sind beispielsweise die im CTFA Cosmetic Ingredient Dictionary unter den Bezeichnungen Polyquaternium beschriebenen Polymere wie Methylvinylimidazoliumchlorid/Vinylpyrrolidon Copolymer (Polyquaternium-16) oder quaternisiertes Vinylpyrrolidon/Dimethylaminoethylmethacrylat Copolymer (Polyquaternium-11) sowie quaternäre Silikonpolymere bzw. -oligomere wie beispielsweise Silikonpolymere mit quaternären Endgruppen (Quaternium-80).

Von den kationischen Polymeren, die in dem erfindungsgemäßen Mittel enhalten sein können, ist zum Beispiel Vinylpyrrolidon/Dimethylaminoethylmethacrylatmethosulfat Copolymer, das unter den Handelsbezeichnungen Gafquat® 755 N und Gafquat® 734 von der Firma Gaf Co., USA vertrieben wird und von denen das Gafquat® 734 besonders bevorzugt ist, geeignet. Weitere kationische Polymere sind beispielsweise das von der Firma BASF, Deutschland unter dem Handelsnamen LUVIQUAT® HM 550 vertriebene Copolymer aus Polyvinylpyrrolidon und Imidazoliminmethochlorid, das von der Firma Calgon/USA unter dem Handelsnamen Merquat® Plus 3300 vertriebene Terpolymer aus Dimethyldiallylammoniumchlorid, Natriumacrylat und Acrylamid, das von der Firma ISP/USA unter dem Handelsnamen Gaffix® VC 713 vertriebene Terpolymer aus Vinylpyrrolidon, Dimethylaminoethylmethacrylat und Vinylcaprolactam, das von der Firma Gaf unter dem Handelsnamen Gafquat® HS 100 vertriebene Vinylpyrrolidon/Methacrylamidopropyltrimethylammoniumchlorid Copolymer und das von der Firma GOLDSCHMIDT/Deutschland unter dem Handelsnamen Abil® Quat 3272 vertriebene diquaternäre Polydimethylsiloxan.

Das erfindungsgemäße Gel enthält als Komponente (E) mindestens eine schaumerzeugende Substanz, beispielsweise ein schaumerzeugendes Polymer oder ein schaumerzeugendes Tensid. Die Tenside können nichtionischen, anionischen oder amphoteren Charakter haben. Bevorzugt sind insbesondere nichtionische, schaumerzeugende Tenside. Die Tenside können einzeln oder in einem Gemisch eingesetzt werden. Die Menge an Tensiden kann variieren und ist so gewählt, daß sich eine ausreichende Menge eines gut in das Haar einarbeitbaren Schaumes bildet, wenn die Zusammensetzung aus der Aerosolverpackung bzw. aus dem Produktspender ausgebracht wird. Die Tensidmenge beträgt typischerweise von 0,01 bis 5 Gewichtsprozent, besonders bevorzugt von 0,1 bis 2 Gewichtsprozent.

Geeignete nichtionische Tenside sind beispielsweise C8-bis C18-Fettalkohole, die mit 8 bis 45 mol Ethylenoxid ethoxyliert sein können, zum Beispiel mit bis zu 40 Mol Ethylenoxid pro Mol Fettalkohol oxethylierter Lauryl-, Tetradecyl-, Cetyl-, Oleyl- oder Stearylalkohol, allein oder im Gemisch; hydriertes, mit 8 bis 45 mol Ethylenoxid ethoxyliertes Rizinusöl, C8- bis C18-Fettsäurealkanolamide; die Fettalkohole von oxethyliertem Lanolin oder oxethyliertes Lanolin; Polyglycerylether von gesättigten oder ungesättigten Fettalkoholen und Alkylphenolen mit 8 bis 30 Kohlenstoffatomen im Alkylrest und 1 bis 10 Glyceryleinheiten im Molekül; Polyethylen/Polypropylen Blockcopolymere und oxethylierte Sorbitanfettsäureester. Besonders bevorzugt als nichtionische Tenside sind außerdem Derivate natürlicher Tenside wie beispielsweise Alkylpolyglykoside.

Geeignete anionische Tenside sind zum Beispiel Alkylsulfate, Alkylethersulfate, Alkylsulfonate, Alkoylsarcosinate, Alkylisethionate oder Dialkylsulfosuccinate wobei die Alkylgruppen 8 bis 18 Kohlenstoffatome enthalten können.

Geeignete amphotere Tenside sind insbesondere solche vom Betaintyp. Beispiele für Betaine umfassen C₈- bis C₁₈-Alkylbetaine, wie Cocodimethylcarboxymethylbetain, Lauryldimethylcarboxymethylbetain, Lauryldimethylalphacarboxyethylbetain, Cetyldimethylcarboxymethylbetain, Oleyldimethylgammacarboxypropylbetain und Laurylbis-(2-hydroxypropyl)alphacarboxyethylbetain; C₈- bis C₁₈-Sulfobetaine wie Cocodimethylsulfopropylbetain, Stearyldimethylsulfopropylbetain, Lauryldimethylsulfoethylbetain, Laurylbis-(2-hydroxyethyl)sulfopropylbetain; die Carboxylderivate des Imidazols, die C₈- bis C₁₈-Alkyldimethylammoniumacetate, die C₈- bis C₁₈-Alkyldimethylcarboxylmethylammoniumsalze sowie die C₈- bis C₁₈-Fettsäurealkylamidobetaine, wie beispielsweise das Kokosfettsäureamidopropylbetain und das N-Kokosfettsäureamidoethyl-N-[2-(carboxymethoxy)ethyl]glycerin (CTFA-Name: Cocoamphocarboxyglycinate).

Das erfindungsgemäße Gel enthält als Komponente (F) mindestens ein Treibmittel. Die Treibmittel können einzeln oder in einem Gemisch eingesetzt werden und sind vorzugsweise in einer Konzentration von 2 bis 40, bevorzugt von 4 bis 20 Gewichtsprozent enthalten. Als Treibmittel sind beispielsweise niedere Alkane, wie zum Beispiel n-Butan, i-Butan und Propan oder deren Gemische sowie Dimethylether und Fluorkohlenwasserstoffe wie F 152 (1,1-Difluorethan) oder F 134 (Tetrafluorethan) sowie ferner bei den in Betracht kommenden Drücken gasförmig vorliegende Treibmittel, wie beispielsweise N₂, N₂O und CO₂ sowie Gemische der vorstehend genannten Treibmittel geeignet.

Das erfindungsgemäße Mittel ist in einer geeigneten, druckdichten Aerosolverpackung verpackt und weist als zusätzliche Komponente eine Vorrichtung zum Verschäumen der Zusammensetzung auf, welche das Verschäumen des Gels unter Verwendung des Treibmittels ermöglicht. Als geeignete Schäumvorrichtung kann beispielsweise ein handelsüblicher Aerosolschaumkopf verwendet werden. Die Verpackung ist vorzugsweise aus einem transparenten Material, durch welches zumindest die Konsistenz, bevorzugt auch die Farbe der Zusammensetzung erkennbar ist.

Das erfindungsgemäße Mittel wird bevorzugt in einem wäßrigen oder in einem wäßrig-alkoholischen Milieu konfektioniert. Als Alkohole können insbesondere die für kosmetische Zwecke üblicherweise verwendeten niederen Alkohole mit 1 bis 4 Kohlenstoffatomen wie zum Beispiel Ethanol und Isopropanol enthalten sein. Der Wassergehalt beträgt vorzugsweise von 35 bis 98, besonders bevorzugt von 55 bis 95 Gewichtsprozent. Der Alkoholgehalt beträgt vorzugsweise von 0 bis 50, besonders bevorzugt von 0,5 bis 30 Gewichtsprozent. Weitere, besonders bevorzugte wasserlösliche Lösungsmittel sind Glycerin und Propylenglykol in einer Menge bis 30 Gewichtsprozent.

Das erfindungsgemäße Mittel kann darüber hinaus die für Haarbehandlungsmittel üblichen Zusatzbestandteile enthalten, zum Beispiel Feuchthaltemittel; Parfümöle in einer Menge von 0,01 bis 5 Gewichtsprozent; Konservierungsmittel, bakterizide und fungizide Wirkstoffe wie zum Beispiel 2,4,4-Trichlor-2-hydroxydiphenylether, Parabene oder Methylchlorisothiazolinon, in einer Menge von 0,01 bis 1,0 Gewichtsprozent; Puffersubstanzen, wie beispielsweise Natriumcitrat oder Natriumphosphat, in einer Menge von 0,1 bis 1,0 Gewichtsprozent; Anfärbestoffe, wie zum Beispiel Fluorescein Natriumsalz, in einer Menge von etwa 0,1 bis 1,0 Gewichtsprozent; Pflegestoffe, wie zum Beispiel Pflanzen- und Kräuterextrakte, Protein- und Seidenhydrolysate, Lanolinderivate, in einer Menge von 0,1 bis 5 Gewichtsprozent; Lichtschutzmittel, Antioxidantien, Radikalfänger, Antischuppenwirkstoffe, Fettalkohole, Glanzgeber, Vitamine, Weichmacher, Kämmbarkeitsverbesserer und rückfettende Agenzien in einer Menge von 0,01 bis 10 Gewichtsprozent.

Das erfindungsgemäße Mittel kann in einem pH-Bereich von 2,0 bis 9,5 vorliegen. Besonders bevorzugt ist der pH-Bereich zwischen 2,5 und 8,5. Liegt das erfindungsgemäße Mittel im sauren Bereich vor, so kann es organische oder anorganische Säuren enthalten wie beispielsweise Ameisensäure, Weinsäure, Äpfelsäure, Maleinsäure, Fumarsäure, Pyrrolidoncarbonsäure, Zitronensäure, Milchsäure, Schwefelsäure, Essigsäure, Salzsäure Phosphorsäure u.a..

Unter Haarbehandlung soll die Behandlung des menschlichen Kopfhaares vor allem zum Zweck der Herstellung einer Frisur oder zur Pflege der Kopfhaare verstanden werden.

Das erfindungsgemäße Mittel wird angewendet, indem eine für den gewünschte Farbeffekt ausreichende Menge in oder auf dem trockenen Haar oder nach der Haarwäsche in oder auf dem feuchten, handtuchgetrockneten Haar verteilt wird. Die anzuwendende Menge hängt von der Haarfülle ab und beträgt typischerweise 1 bis 25 g. Das Mittel wird vorzugsweise nicht ausgespült, verbleibt also auf dem Haar. Anschließend wird das Haar gegebenenfalls durchgekämmt oder zur Frisur geformt und gegebenenfalls getrocknet. Die Frisurenerstellung kann aber auch bereits vor der Aufbringung des Mittels erfolgen, beispielsweise wenn anschließend nur die Lockenspitzen, Haarspitzen oder einzelne Strähnen farbig gestaltet werden sollen.

Die nachfolgenden Beispiele sollen den Gegenstand der Erfindung näher erläutern.

### Beispiele

### Beispiel 1: Color Mousse

| | |
|---|---|
| 6,00 g | Dichrona® BG (Merck) ¹⁾ |
| 4,00 g | Timiron® Starluster MP 115 (Merck) ²⁾ |
| 2,00 g | Polyvinylpyrrolidon |
| 1,80 g | 1,2-Propylenglykol |
| 0,22 g | Decylglucosid |
| 0,20 g | Styrol/PVP Copolymer |
| 0,20 g | p-Hydroxybenzoesäuremethylester |
| 0,20 g | Polyacrylsäure (Carbomer) |
| 0,18 g | PEG-40 Hydrogenated Castor Oil |
| 0,15 g | Aminomethylpropanol |
| 20,00 g | Ethanol |
| 10,00 g | Propan/Butan 2,7 |
| | Wasser ad 100 g |

| | |
|---|---|
| ¹⁾ Titandioxid/Mica/Ferric Ferrocyanide-Pigment, blauglänzend mit Grünschimmer | |
| ²⁾ Mica/Titandioxid-Pigment, silberglänzend | |

Das Mittel liegt als Flüssiggel vor, welches in einer durchsichtigen PET-Verpackung mit einem Schaumventil verpackt ist. Die Pigmente sind stabil dispergiert und setzen sich auch bei längeren Standzeiten (mehrere Monate) nicht ab. Das Mittel läßt sich sehr gut verschäumen. Bei Ausbringung des Mittel bildet sich ein stabiler, farbiger, voluminöser Schaum, der nicht vom Haar abläuft, bei Einarbeitung in das Haar aber leicht zusammenbricht.

### Beispiel 2: Color Mousse

| | |
|---|---|
| 10,0 g | Colorona Majestic Green® (Merck) ¹⁾ |
| 1,0 g | Timiron® Starluster MP 115 (Merck) ²⁾ |
| 1,0 g | Polyvinylpyrrolidon |
| 1,0 g | Polyvinylpyrrolidon/Vinylacetat Copolymer |
| 0,5 g | Decylglucosid |
| 0,4 g | Phenoxyethanol |
| 6,3 g | Pemulen® TR1 (BF Goodrich Chemical) ³⁾ |
| 0,1 g | Hydroxyethylcellulose |
| 0,075 g | Aminomethylpropanol |
| 10,0 g | Ethanol |
| 10,00 g | Propan/Butan 2,7 |
| | Wasser ad 100 g |

| | |
|---|---|
| ¹⁾ Titandioxid/Mica/Chromoxid Grün-Pigment, grünglänzend | |
| ²⁾ Mica/Titandioxid-Pigment, silberglänzend | |
| ³⁾ Acrylates/C10-30 Alkyl Acrylates Crosspolymer | |

## Patentansprüche

1. Verschäumbares, farbiges Gel mit einem Gehalt an
(A) 35 bis 98 Gewichtsprozent Wasser,
(B) mindestens einem gelbildenden Verdicker, ausgewählt unter Verdickern, die der Zusammensetzung eine Fließgrenze verleihen,
(C) mindestens einem Pigment ausgewählt aus organischen Pigmenten, anorganisch-organischen Mischpigmenten, anorganischen Pigmenten natürlichen Ursprungs, Weißpigmenten, Schwarzpigmenten, Fluoreszenzpigmenten, Phosphoreszenzpigmenten, farbigen Metalloxiden, farbigen Metallhydroxiden, farbigen Metalloxidhydraten, farbigen Mischphasenpigmenten, farbigen schwefelhaltigen Silicaten, farbigen Metallsulfiden, farbigen komplexen Metallcyaniden, farbigen Metallsulfaten, farbigen Metallchromaten, farbigen Metallmolybdaten, Bronzepigmenten, schwarzem Eisenoxid, gelbem Eisenoxid, rotem Eisenoxid, braunem Eisenoxid, Manganviolett, Ultramarin, Chromoxidhydrat, Eisenblau, Carmine und Pigmenten auf Mica- oder Glimmerbasis, welche mit einem Metalloxid oder einem Metalloxychlorid sowie mit einem farbgebenden Stoff, ausgewählt aus Eisenoxiden, Eisenblau, Ultramarin, Carmin und Chromoxid beschichtet sind,
(D) mindestens einem filmbildenden, haarfestigenden Polymer,
(E) mindestens einem schaumerzeugenden Tensid und
(F) mindestens einem Treibmittel
in Kombination mit einer geeigneten Aerosolverpackung und einer Vorrichtung zum Verschäumen des Gels, wobei die Konzentration des Verdickers so gewählt ist, daß einerseits die Viskosität des Gels gering genug ist, um ein Verschäumen des Gels zu ermöglichen und andererseits die Fließgrenze mindestens so groß ist, daß ein Absinken der Pigmente verhindert ist, ausgenommen einer Zusammensetzung enthaltend (a) Titandioxid beschichtetes Mica, wobei Titandioxid beschichtetes Mica mit einer Teilchengröße von 20 µm oder größer nicht mehr als 10 Volumen% bezogen auf das gesamte Volumen von Titandioxid beschichtetem Mica ausmacht, (b) ein Carboxyvinylpolymer und (c) ein amphoteres Makromolekül.

2. Gel nach Anspruch 1, **dadurch gekennzeichnet, daß** es als Pigmente anorganische Pigmente enthält.

3. Gel nach einem der vorhergehenden Ansprüche, **dadurch gekennzeichnet, daß** die Verdicker ausgewählt sind aus vernetzter oder unvernetzter Polyacrylsäure oder Polymethacrylsäure.

4. Gel nach einem der vorhergehenden Ansprüche, **dadurch gekennzeichnet, daß** es Komponente (B) in einer Konzentration von 0,05 bis 10 Gew.% enthält.

5. Gel nach einem der vorhergehenden Ansprüche, **dadurch gekennzeichnet, daß** es Komponente (C) in einer Konzentration von 0,01 bis 25 Gew.% enthält.

6. Gel nach einem der vorhergehenden Ansprüche, **dadurch gekennzeichnet, daß** es Komponente (D) in einer Konzentration von 0,01 bis 25 Gew.% enthält.

7. Gel nach einem der vorhergehenden Ansprüche, **dadurch gekennzeichnet, daß** es Komponente (E) in einer Konzentration von 0,01 bis 5 Gew.% enthält.

8. Gel nach einem der vorhergehenden Ansprüche, **dadurch gekennzeichnet, daß** die Aerosolverpackung transparent ist.

9. Verwendung des Gels gemäß einem der vorgehenden Ansprüche zur Erzielung von temporären Farbeffekten auf Haaren.

## Claims

1. Foamable coloured gel with a content of
(A) 35 to 98% by weight of water,
(B) at least one gel-forming thickener chosen from thickeners which give the composition a yield point,
(C) at least one pigment chosen from organic pigments, inorganic-organic mixed pigments, inorganic pigments of natural origin, white pigments, black pigments, fluorescent pigments, phosphorescent pigments, coloured metal oxides, coloured metal hydroxides, coloured metal oxide hydrates, coloured mixed-phase pigments, coloured sulphur-containing silicates, coloured metal sulphides, coloured complex metal cyanides, coloured metal sulphates, coloured metal chromates, coloured metal molybdates, bronze pigments, black iron oxide, yellow iron oxide, red iron oxide, brown iron oxide, manganese violet, ultramarine, chromium oxide hydrate, iron blue, carmines and pigments based on mica which have been coated with a metal oxide or a metal oxychloride, and with a colouring substance chosen from iron oxides, iron blue, ultramarine, carmine and chromium oxide,
(D) at least one film-forming, hair-setting polymer,
(E) at least one foam-generating surfactant and
(F) at least one propellant
in combination with a suitable aerosol packaging and a device for foaming the gel, where the concentration of the thickener is chosen such that, on the one hand, the viscosity of the gel is low enough to permit foaming of the gel and, on the other hand, the yield point is at least high enough to prevent the pigments from settling out, with the exception of a composition comprising (a) titanium dioxide-coated mica, where titanium dioxide-mica with a particle size of 20 µm or above constitutes not more than 10% by volume, based on the total volume of titanium dioxide-coated mica, (b) a carboxyvinyl polymer and (c) an amphoteric macromolecule.

2. Gel according to Claim 1, **characterized in that** it comprises inorganic pigments as pigment.

3. Gel according to one of the preceding claims, **characterized in that** the thickeners are chosen from crosslinked or uncrosslinked polyacrylic acid or polymethacrylic acid.

4. Gel according to one of the preceding claims, **characterized in that** it comprises component (B) in a concentration of from 0.05 to 10% by weight.

5. Gel according to one of the preceding claims, **characterized in that** it comprises component (C) in a concentration of from 0.01 to 25% by weight.

6. Gel according to one of the preceding claims, **characterized in that** it comprises component (D) in a concentration of from 0.01 to 25% by weight.

7. Gel according to one of the preceding claims, **characterized in that** it comprises component (E) in a concentration of from 0.01 to 5% by weight.

8. Gel according to one of the preceding claims, **characterized in that** the aerosol packaging is transparent.

9. Use of the gel as in one of the preceding claims for achieving temporary colour effects on hair.

## Revendications

1. Gel moussant coloré contenant
(A) de 35 à 98 % en poids d'eau,
(B) au moins un épaississant gélifiant choisi parmi des épaississants qui confèrent une limite d'écoulement à la composition,
(C) au moins un pigment choisi parmi des pigments organiques, des pigments mixtes organiques-minéraux, des pigments minéraux d'origine naturelle, des pigments blancs, des pigments noirs, des pigments fluorescents, des pigments phosphorescents, des oxydes métalliques colorés, des hydroxydes métalliques colorés, des hydrates métalliques colorés, des pigments en phases mixtes colorés, des silicates soufrés colorés, des sulfures métalliques colorés, des cyanures métalliques complexes colorés, des sulfates métalliques colorés, des chromates métalliques colorés, des molybdates métalliques colorés, des pigments bronze, l'oxyde de fer noir, l'oxyde de fer jaune, l'oxyde de fer rouge, l'oxyde de fer brun, le violet de manganèse, l'outremer, l'hydrate de chrome, le bleu de fer, les carmins et des pigments à base de mica qui sont enrobés d'un oxyde métallique ou d'un oxychlorure métallique ainsi que d'une substance colorante choisie parmi les oxydes de fer, le bleu de fer, l'outremer, le carmin et l'oxyde de chrome.
(D) au moins un polymère filmogène fixant les cheveux,
(E) au moins un tensioactif générateur de mousse et
(F) au moins un propulseur
en association avec un conditionnement pour aérosol approprié et un dispositif destiné à faire mousser le gel, la concentration de l'épaississant étant choisie de manière que, d'une part, la viscosité du gel soit suffisamment faible pour permettre le moussage du gel et, d'autre part, la limite d'écoulement soit au moins suffisamment élevée pour empêcher une sédimentation des pigments,
à l'exclusion d'une composition contenant (a) du mica enrobé de dioxyde de titane, le mica enrobé de dioxyde de titane ayant une taille de particules de 20 µm ou plus ne représentant pas plus de 10 % en volume par rapport au volume total du mica enrobé de dioxyde de titane, (b) un polymère carboxyvinylique et (c) une macromolécule amphotère.

2. Gel selon la revendication 1, **caractérisé en ce qu'**il contient comme pigments des pigments minéraux.

3. Gel selon l'une quelconque des revendications précédentes, **caractérisé en ce que** les épaississants sont choisis parmi un poly(acide acrylique) ou poly(acide méthacrylique) réticulé ou non réticulé.

4. Gel selon l'une quelconque des revendications précédentes, **caractérisé en ce qu'**il contient le composant (B) à une concentration de 0,05 à 10 % en poids.

5. Gel selon l'une quelconque des revendications précédentes, **caractérisé en ce qu'**il contient le composant (C) à une concentration de 0,01 à 25 % en poids.

6. Gel selon l'une quelconque des revendications précédentes, **caractérisé en ce qu'**il contient le composant (D) à une concentration de 0,01 à 25 % en poids.

7. Gel selon l'une quelconque des revendications précédentes, **caractérisé en ce qu'**il contient le composant (E) à une concentration de 0,01 à 5 % en poids.

8. Gel selon l'une quelconque des revendications précédentes, **caractérisé en ce que** le conditionnement pour aérosol est transparent.

9. Utilisation du gel selon l'une quelconque des revendications précédentes, pour atteindre des effets de teinture temporaire sur des cheveux.
